(19) Europäisches Patentamt
European Patent Office
Office européen des brevets

(11) Veröffentlichungsnummer: **0 564 920 A1**

(12) **EUROPÄISCHE PATENTANMELDUNG**

(21) Anmeldenummer: **93104985.2**

(22) Anmeldetag: **25.03.93**

(51) Int. Cl.5: **C07D 239/34**, C07D 239/60, C07D 251/30, A01N 43/54, A01N 43/66

(30) Priorität: **07.04.92 DE 4211610**

(43) Veröffentlichungstag der Anmeldung:
**13.10.93 Patentblatt 93/41**

(84) Benannte Vertragsstaaten:
**BE CH DE DK FR GB IT LI NL**

(71) Anmelder: **BAYER AG**
**D-51368 Leverkusen(DE)**

(72) Erfinder: **Andree, Roland, Dr.**
**Dechant-Miebach-Weg 37**
**W-4018 Langenfeld(DE)**
Erfinder: **Drewes, Mark Wilhelm, Dr.**
**Talstrasse 54**
**W-4018 Langenfeld(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch-Gladbach 2(DE)**
Erfinder: **Schmidt, Robert R., Dr.**
**Im Waldwinkel 110**
**W-5060 Bergisch Gladbach 2(DE)**

(54) **Substituierte Azine.**

(57) Die vorliegende Erfindung betrifft neue substituierte Azine, der allgemeinen Formel (I),

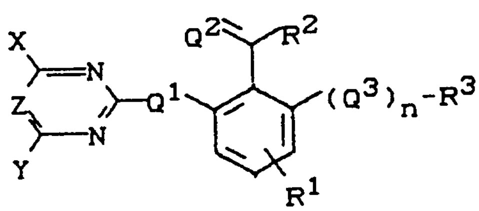

(I)

in welcher
n, $Q^1$, $Q^2$, $Q^3$, $R^1$, $R^2$, $R^3$, X, Y und Z die in der Beschreibung angegebenen Bedeutungen haben, mehrere Verfahren zu ihrer Herstellung, sowie ihre Verwendung als Herbizide.

EP 0 564 920 A1

Die Erfindung betrifft neue substituierte Azine, mehrere Verfahren zu ihrer Herstellung sowie ihre Verwendung als Herbizide.

Eine Reihe von substituierten Azinen mit herbiziden Eigenschaften ist bereits bekannt (vergleiche JP-A 54117486 - Zit. in Chem. Abstracts 93, 150268c; EP-A 223 406; EP-A 249 708; JP-A 63258467 - Zit. in Chem. Abstracts 110, 130532a; JP-A 63258463 - Zit. in Chem. Abstracts 110, 192853q; EP-A 287 079; EP-A 374 839; EP-A 008 192; EP-A 321 846; EP-A 336 494; EP-A 459 243; DE-OS 4 030 041). Verbindungen aus den genannten Publikationen haben jedoch bisher keine größere Bedeutung erlangt.

Es wurden nun die neuen substituierten Azine der allgemeinen Formel (I) gefunden,

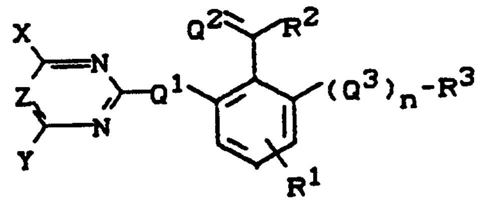

(I)

in welcher

n für die Zahlen 0 oder 1 steht,

$Q^1$ und $Q^3$ gleich oder verschieden sind und für Sauerstoff, Schwefel, NH oder N-Alkyl stehen,

$Q^2$ für Sauerstoff, Schwefel oder eine der nachstehenden Gruppierungen steht:
$N-R^4$ oder

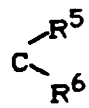

$R^1$ für Wasserstoff, Amino, Hydroxy, Cyano, Nitro, Halogen oder für einen Rest der Reihe Alkyl, Halogenalkyl, Alkoxy, Halogenalkoxy` Alkylthio, Halogenalkylthio, Alkylamino, Dialkylamino, Alkylcarbonylamino, Alkoxycarbonylamino, Alkylsulfonylamino oder Phenyl steht,

$R^2$ für Wasserstoff, Hydroxy, Alkyl oder für einen jeweils gegebenenfalls substituierten Rest der Reihe Alkoxy, Alkylthio, Alkylamino, Dialkylamino, Aralkyloxy, Aralkylthio, Aralkylamino, Aryloxy, Arylthio oder Arylamino steht,

$R^3$ für durch Cyano, Carboxy oder Alkoxycarbonyl substituiertes Alkyl mit mindestens zwei Kohlenstoffatomen oder für gegebenenfalls durch Cyano, Carboxy, Halogen, Alkoxycarbonyl, Aryl (welches gegebenenfalls durch Halogen, Alkyl oder Alkoxy substituiert ist) oder Heteroaryl substituiertes Alkenyl steht,

$R^4$ für Wasserstoff, Amino oder für einen jeweils gegebenenfalls substituierten Rest der Reihe Alkyl, Alkenyl, Alkinyl, Aralkyl, Aryl, Alkoxycarbonyloxy, Arylaminocarbonyloxy, Carboxyalkoxy, Alkoxycarbonylalkoxy, Alkylamino, Dialkylamino, Aralkylamino, Arylamino, N-Alkyl-N-arylamino, Alkylcarbonylamino, Arylcarbonylamino, Heteroarylamino, Heteroarylcarbonylamino, Alkoxycarbonylamino, Alkylsulfonylamino oder Arylsulfonylamino steht,

$R^5$ für Wasserstoff, Halogen, Cyano, Carboxy, Alkoxycarbonyl, Alkylcarbonylamino oder Dialkoxyphosphoryl steht,

$R^6$ für Formyl, Cyano, Carboxy, Hydroxymethyl, Carbamoyl oder für einen jeweils gegebenenfalls substituierten Rest der Reihe Alkoxycarbonyl, Cycloalkyloxycarbonyl, Aralkoxycarbonyl, Aryloxycarbonyl, Heterocyclylalkoxycarbonyl, Alkylthiocarbonyl, Aralkylthiocarbonyl, Arylthiocarbonyl, Alkylaminocarbonyl, Cycloalkylaminocarbonyl, Aralkylaminocarbonyl, Arylaminocarbonyl, Dialkylaminocarbonyl, Arylaminocarbonylalkoxycarbonyl, Dialkylaminocarbonylalkoxycarbonyl, Alkylaminocarbonylalkoxycarbonyl, Alkylhydrazinocarbonyl, Arylhydrozinocarbonyl, Pyrrolidinylcarbonyl, Piperidinylcarbonyl, Morpholinylcarbonyl, Piperazinylcarbonyl oder Phthalimidoxycarbonyl steht,

X und Y gleich oder verschieden sind und für Wasserstoff, Halogen oder einen Rest der Reihe Alkyl, Halogenalkyl, Alkoxy, Halogenalkoxy, Alkylthio, Alkylamino oder Dialkylamino stehen

und

Z für Stickstoff oder eine CH- oder C-Halogen-Gruppierung steht.

Man erhält die neuen substituierten Azine der allgemeinen Formel (I), wenn man

(a) Azinverbindungen der allgemeinen Formel (II)

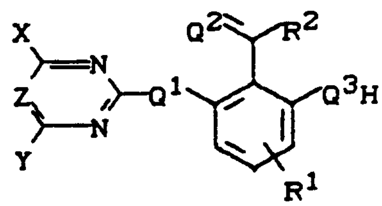

(II)

in welcher

$Q^1$, $Q^2$, $Q^3$, $R^1$, $R^2$, X, Y und Z die oben angegebenen Bedeutungen haben,

mit Halogenverbindungen der allgemeinen Formel (III)

$X^1$-$R^3$ (III)

in welcher

$R^3$ die oben angegebenen Bedeutungen hat und

$X^1$ für Halogen steht,

gegebenenfalls In Gegenwart eines Säureakzeptors und gegebenenfalls in Gegenwart eines Verdünnungsmittels umsetzt, oder wenn man

(b) Benzolderivate der allgemeinen Formel (IV)

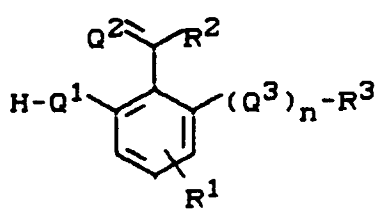

(IV)

in welcher

n, $Q^1$, $Q^2$, $Q^3$, $R^1$, $R^2$ und $R^3$ die oben angegebenen Bedeutungen haben,

mit reaktionsfähigen Azinen der allgemeinen Formel (V)

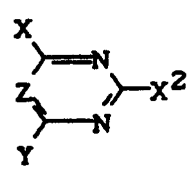

(V)

in welcher

X, Y und Z die oben angegebene Bedeutung haben und

$X^2$ für eine nucleophile Abgangsgruppe steht,

gegebenenfalls in Gegenwart eines Säureakzeptors und gegebenenfalls in Gegenwart eines Verdünnungsmittels umsetzt, oder wenn man

(c) Carbonylverbindungen der allgemeinen Formel (Ia)

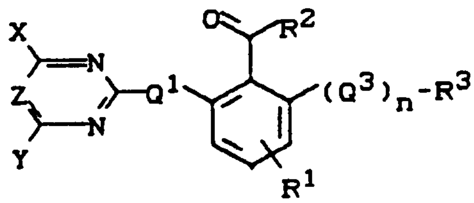

(Ia)

in welcher
n, $Q^1$, $Q^3$, $R^1$, $R^2$, $R^3$, X, Y und Z die oben angegebenen Bedeutungen haben,
mit Aminoverbindungen der allgemeinen Formel (VI)

$H_2N-R^4$ (VI)

in welcher
$R^4$ die oben angegebenen Bedeutungen hat,
oder mit Methylenverbindungen der allgemeinen Formel (VII)

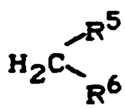

(VII)

in welcher
$R^5$ und $R^6$ die oben angegebenen Bedeutungen haben,
gegebenenfalls in Gegenwart eines Reaktionshilfsmittels und gegebenenfalls in Gegenwart eines Verdünnungsmittels umsetzt.

Die neuen substituierten Azine der allgemeinen Formel (I) zeichnen sich durch starke Herbizidwirkung aus.

Die Erfindung betrifft vorzugsweise Verbindungen der Formel (I), in welcher
n für die Zahlen 0 oder 1 steht,
$Q^1$ und $Q^3$ gleich oder verschieden sind und für Sauerstoff, Schwefel, NH oder N-$C_1$-$C_4$-Alkyl stehen,
$Q^2$ für Sauerstoff, Schwefel oder eine der nachstehenden Gruppierungen steht:
N-$R^4$ oder

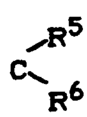

$R^1$ für Wasserstoff, Amino, Hydroxy, Cyano, Nitro, Halogen oder für einen Rest der Reihe $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Di-($C_1$-$C_4$-alkyl)-amino, $C_1$-$C_4$-Alkyl-carbonylamino, $C_1$-$C_4$-Alkoxy-carbonylamino, $C_1$-$C_4$-Alkylsulfonylamino oder Phenyl steht,
$R^2$ für Wasserstoff, Hydroxy, $C_1$-$C_4$-Alkyl oder für einen jeweils gegebenenfalls substituierten Rest der Reihe $C_1$-$C_6$-Alkoxy, $C_1$-$C_6$-Alkylthio, $C_1$-$C_6$-Alkylamino, Di-($C_1$-$C_4$-alkyl)-amino, Phenyl-$C_1$-$C_2$-alkyloxy, Phenyl-$C_1$-$C_2$-alkylthio, Phenyl-$C_1$-$C_2$-alkylamino, Phenoxy, Phenylthio oder Phenylamino steht, wobei die möglichen Substituenten in den Alkylteilen vorzugsweise ausgewählt sind aus der Reihe Fluor, Chlor, Cyano, Nitro, Carboxy, $C_1$-$C_4$-Alkoxy, $C_1$-$C_4$-Alkoxy-carbonyl, und die möglichen Substituenten in den Arylteilen vorzugsweise ausgewählt sind aus der Reihe Fluor, Chlor, Brom, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl,
$R^3$ für durch Cyano, Carboxy oder $C_1$-$C_4$-Alkoxy-carbonyl substituiertes $C_2$-$C_6$-Alkyl oder für gegebenenfalls durch Cyano, Carboxy, Halogen, $C_1$-$C_4$-Alkoxy-carbonyl,Phenyl (welches gegebenenfalls durch Halogen, $C_1$-$C_4$-Alkyl oder $C_1$-$C_4$-Alkoxy substituiert ist), Naphthyl,

Thienyl, Furyl oder Pyridyl substituiertes $C_2$-$C_6$-Alkenyl steht,

$R^4$ für Wasserstoff, Amino oder für einen jeweils gegebenenfalls durch Halogen substituierten Rest der Reihe $C_1$-$C_6$-Alkyl, $C_3$-$C_6$-Alkenyl, $C_3$-$C_6$-Alkinyl, Carboxy-$C_1$-$C_2$-alkoxy, $C_1$-$C_6$-Alkoxycarbonyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkyl-carbonylamino, $c_1$-$c_6$-Alkoxy-carbonylamino, $C_1$-$C_6$-Alkyl-sulfonylamino, oder für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenylaminocarbonyloxy, Phenylamino, Phenyl-$C_1$-$C_4$-alkylamino, N-($C_1$-$C_4$-alkyl)-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^5$ für Wasserstoff, Halogen, Cyano, Carboxy, $C_1$-$C_6$-Alkoxy-carbonyl, $C_1$-$C_6$-Alkylcarbonylamino oder Di-($C_1$-$C_4$-alkoxy)-phosphoryl steht,

$R^6$ für Formyl, Cyano, Carboxy, Hydroxrmethyl, Carbamoyl oder für einen jeweils gegebenenfalls durch Halogen, Carboxy oder $C_1$-$C_4$-Alkoxy-carbonyl substituierten Rest der Reihe $C_1$-$C_6$-Alkoxy-carbonyl, $C_5$-$C_6$-Cycloalkyloxy-carbonyl, $C_1$-$C_6$-Alkylthio-carbonyl, $C_1$-$C_6$-Alkylamino-carbonyl, $C_5$-$C_6$-Cycloalkylamino-carbonyl, Di-($C_1$-$C_2$-alkyl)-aminocarbonyl, $C_1$-$C_4$-Alkylamino-carbonyl-$C_1$-$C_4$-alkoxy-carbonyl, Di-($C_1$-$C_2$-alkyl)-amino-carbonyl-$C_1$-$C_4$-alkoxy-carbonyl, Phenylaminocarbonyl-$C_1$-$C_4$-alkoxy-carbonyl, N-Methyl-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthiocarbonyl, Phenylaminocarbonyl, Phenyl-$C_1$-$C_4$-alkylamino-carbonyl, N-($C_1$-$C_4$-Alkyl)-phenylamino-carbonyl oder Phenylhydrazinocarbonyl oder für Phthalimidoxycarbonyl steht,

X und Y gleich oder verschieden sind und für Wasserstoff, Halogen oder einen Rest der Reihe $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen und

Z für Stickstoff oder eine CH oder C-Halogen-Gruppierung steht.

Die in der Definition der erfindungsgemäßen Verbindungen aufgeführten aliphatischen Kohlenwasserstoffreste (z.B. Alkyl, Alkenyl, Alkinyl), auch in Kombination mit Heteroatomen (z.B. in Alkoxy, Alkylthio, Alkylamino), sind jeweils geradkettig oder verzweigt. Halogen steht jeweils im allgemeinen für Fluor, Chlor, Brom oder Jod, vorzugsweise für Fluor, Chlor oder Brom, insbesondere für Fluor oder Chlor.

Die Erfindung betrifft insbesondere Verbindungen der Formel (I), in welcher

n für die Zahlen 0 oder 1 steht,

$Q^1$ und $Q^3$ jeweils für Sauerstoff stehen,

$Q^2$ für Sauerstoff oder eine der nachstehenden Gruppierungen steht:

N-$R^4$ oder

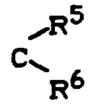

$R^1$ für Wasserstoff, Amino, Hydroxy, Fluor, Chlor, Brom, Methyl, Trifluormethyl, Methoxy, Difluormethoxy, Trifluormethoxy, Methylthio, Trifluormethylthio, Methylamino, Dimethylamino, Acetylamino, Methoxycarbonylamino, Methylsulfonylamino oder Phenyl steht,

$R^2$ für Wasserstoff, Hydroxy, Methyl oder für jeweils gegebenenfalls substituiertes Methoxy, Ethoxy, Propoxy, Butoxy, Benzyloxy, Phenylethoxy oder Phenoxy steht, wobei die möglichen Substituenten In den Alkylteilen vorzugsweise ausgewählt sind aus der Reihe Fluor, Chlor, Cyano, Carboxy, Methoxy, Ethoxy, Methoxycarbonyl, Ethoxycarbonyl, und die möglichen Substituenten in den Arylteilen vorzugsweise ausgewählt sind aus der Reihe

Fluor, Chlor, Brom, Methyl, Trifluormethyl, Methoxy, Trifluormethoxy, Methoxycarbonyl und Ethoxycarbonyl,

$R^3$ für durch Cyano, Carboxy, Methoxycarbonyl oder Ethoxycarbonyl substituiertes Ethyl, Propyl oder Butyl oder für gegebenenfalls durch Cyano, Carboxy, Fluor, Chlor, Brom, Methoxycarbonyl, Ethoxycarbonyl, Phenyl (welches gegebenenfalls durch Fluor, Chlor, Brom, Methyl oder Methoxy substituiert ist), Naphthyl, Thienyl, Furyl oder Pyridyl substituiertes Ethenyl, Propenyl oder Butenyl steht,

$R^4$ für Wasserstoff, Amino, für Methyl, Ethyl, Propyl, Isopropyl, Butyl, Isobutyl, sec-Butyl, tert-Butyl, Allyl, Propargyl, Carboxymethoxy, Carboxyethoxy, Methoxycarbonyloxy, Ethoxycarbonyloxy, Propoxycarbonyloxy, Butoxycarbonyloxy, Methoxycarbonylmathoxy, Ethoxycarbonylmethoxy, Methoxycarbonylothoxy, Ethoxycarbonylethoxy, Methylamino, Ethylamino, Propylamino, Isopropylamino, Butylamino, laobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jaweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenyl, Benzyl, Phenylaminocarbonyloxy, Phenylamino, Benzylamino, N-Methylphenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^5$ für Wasserstoff, Fluor, Chlor, Cyano, Carboxy, $C_1$-$C_4$-Alkoxy-carbonyl, $C_1$-$C_4$-Alkylcarbonylamino, Dimethoxyphosphoryl oder Diethoxyphosphoryl steht,

$R^6$ für Formyl, Cyano, Carboxy, Hydroxymethyl, Carbamoyl, für einen jeweils gegebenenfalls durch Fluor, Chlor, Carboxy oder $C_1$-$C_4$-Alkoxy-corbonyl substituierten Rest der Reihe $C_1$-$C_4$-Alkoxy-carbonyl, $C_5$-$C_6$-Cycloalkyloxy-carbonyl, $C_1$-$C_4$-Alkylthiocarbonyl, $C_1$-$C_4$-Alkylaminocarbonyl, $C_5$-$C_6$-Cycloalkylamino-carbonyl, Dimethylaminocarbonyl, $C_1$-$C_4$-Alkylaminocarbonyl-$C_1$-$C_4$-alkoxy-carbonyl, für Dimethylaminocarbonyl-$C_1$-$C_4$-alkoxy-carbonyl, für N-Methyl-phenylaminocarbonyl-$C_1$-$C_4$-alkoxy-carbonyl, für jeweils gegebenenfalls durch Methyl und/oder Ethyl substituiertes Pyrrolidinyl-carbonyl, Piperidinyl-carbonyl, Morpholinyl-carbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-phenylaminocarbonyl, Phenylhydrazinocarbonyl oder für Phthalimidoxycarbonyl steht,

X und Y gleich oder verschieden sind und für Wasserstoff, Chlor, Methyl, Ethyl, Trifluormethyl, Methoxy, Ethoxy, Difluormethoxy, Methylthio, Methylamino, Ethylamino oder Dimethylamino stehen und

Z für Stickstoff oder eine CH-Gruppierung steht.

Verwendet man für das erfindungsgemäße Verfahren (a) beispielsweise 2-Hydroxy-6-(4,6-dimethoxy-pyrimidin-2-yl-oxy)-benzaldehyd und $\beta$-Chlorpropionitril als Ausgangsstoffe, so kann der Reaktionsablauf durch das folgende Formelschema wiedergegeben werden:

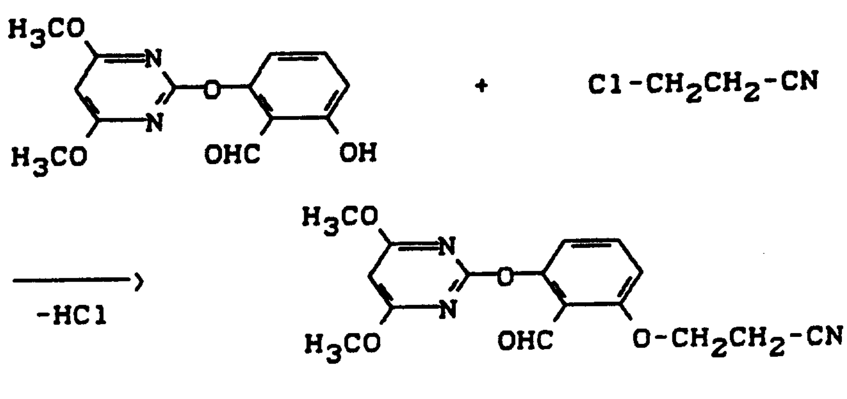


Verwendet man für das erfindungsgemäße Verfahren (b) beispielsweise 2-Hydroxy-6-styryl-benzoesäureethylester und 2-Chlor-4,6-dimethoxy-s-triazin als Ausgangsstoffe, so kann der Reaktionsablauf durch das folgende Formelschema wiedergegeben werden:

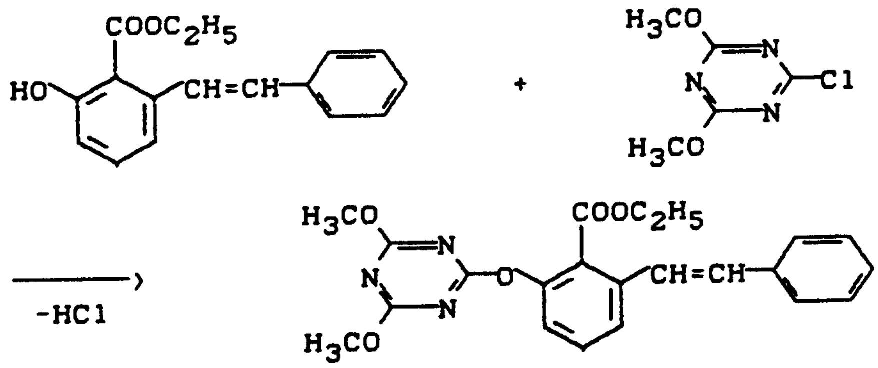


Verwendet man für das erfindungsgemäße Verfahren (c) beispielsweise 2-(1-Methoxycarbonyl-ethoxy)6-(4,6-dimethoxy-pyrimidin-2-yl-oxy)-acetophenon und Methansulfonsäurehydrazid als Ausgangsstoffe, so kann der Reaktionsablauf durch das folgende Formelschema wiedergegeben werden:

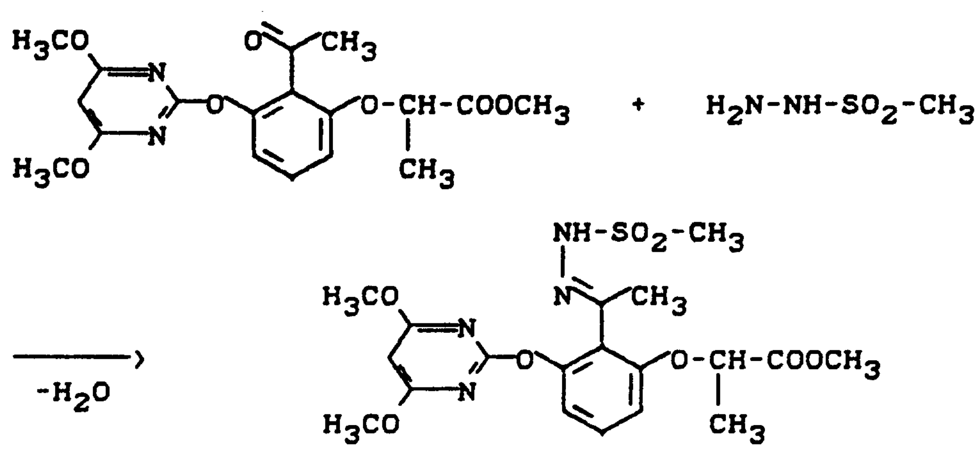


Die beim erfindungsgemäßen Verfahren (a) zur Herstellung von Verbindungen der Formel (I) als Ausgangsstoffe zu verwendenden Azinverbindungen sind durch die Formel (II) allgemein definiert.

In Formel (II) haben $Q^1$, $Q^2$, $Q^3$, $R^1$, $R^2$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $Q^1$, $Q^2$, $Q^3$, $R^1$, $R^2$, X, Y und Z angegeben wurden.

Als Beispiele für die Ausgangsstoffe der Formel (II) seien genannt:
2-Hydroxy-6-(4,6-dimethoxy-pyrimidin-2-yl-oxy)-benzaldehyd, -benzoesäure, -benzoesäure-methylester und -benzoesäureethylester.

Die Ausgangsstoffe der Formel (II) sind bekannt und/oder können nach an sich bekannten Verfahren hergestellt werden (vgl. EP-A 249 708, EP-A 321 846, EP-A 336 494, DE-OS 4 030 041).

Die beim erfindungsgemäßen Verfahren (a) weiter als Ausgangsstoffe zu verwendenden Halogenverbindungen sind durch die Formel (III) allgemein definiert.

In Formel (III) hat $R^3$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^3$ angegeben wurde; $X^1$ steht vorzugsweise für Chlor, Brom oder Iod.

Als Beispiele für die Ausgangsstoffe der Formel (III) seien genannt:
$\beta$-Chlor-(bzw. Brom-)-propionitril, $\gamma$-Chlor-(bzw. Brom-)-butyronitril, $\alpha$- und $\beta$-Chlor-(bzw. Brom-)-propionsäure sowie deren Methylester und Ethylester, $\alpha$-, $\beta$- und $\gamma$-Chlor-(bzw. Brom-)-buttersäure sowie deren Methylester und Ethylester.

Die Ausgangsstoffe der Formel (III) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische

und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliumtert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden, Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Die beim erfindungsgemäßen Verfahren (b) zur Herstellung von Verbindungen der Formel (I) als Ausgangsstoffe zu verwendenden Benzolderivate sind durch die Formel (IV) allgemein definiert.

In Formel (IV) haben n, $Q^1$, $Q^2$, $Q^3$, $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $Q^1$, $Q^2$, $Q^3$, $R^1$, $R^2$ und $R^3$ angegeben wurden.

Als Beispiele für die Ausgangsstoffe der Formel (IV) seien genannt:

2-Hydroxy-6-styryl-benzaldehyd und -benzoesäure sowie deren Methylester und Ethylester.

Die Ausgangsstoffe der Formel (IV) sind bekannt und/oder können nach an sich bekannten Verfahren hergestellt werden (vgl. Synthesis 1988, 525-529).

Die beim erfindungsgemäßen Verfahren (b) weiter als Ausgangsstoffe zu verwendenden reaktionsfähigen Azine sind durch die Formel (V) allgemein definiert.

In Formel (V) haben X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X, Y und Z angegeben wurden;

$X^2$ steht vorzugsweise für Fluor, Chlor, Brom oder $C_1$-$C_4$-Alkylsulfonyl, insbesondere für Chlor oder Methylsulfonyl.

Als Beispiele für die Ausgangsstoffe der Formel (V) seien genannt:

2-Chlor- und 2-Methylsulfonyl-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidin, -4-chlor-6-methyl-pyrimidin, -4-trifluormethyl-6-methoxy-pyrimidin, -4-methoxy-6-difluormethoxy-pyrimidin, -4-methyl-6-difluormethoxy-pyrimidin, -4,6-bis-difluormethoxy-pyrimidin, -4-chlor-6-ethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-dimethoxy-pyrimidin, -4-methoxy-6-methylamino-pyrimidin und -4,6-bis-trifluormethyl-pyrimidin, sowie 2-Chlor-4,6-dimethyl-s-triazin, -4-methoxy-6-methyl-s-triazin, -4,6-dimethoxy-s-triazin, -4-ethoxy-6-methyl-s-triazin und -4-ethyl-6-methoxy-s-triazin.

Die reaktionsfähigen Azine der Formel (V) sind bekannt und/oder können nach an sich bekannten Verfahren hergestellt werden (vgl. J. Chem. Soc. 1957, 1830, 1833; J. Org. Chem. 26 (1961), 792; US-P 3308119; US-P 4711959).

Das erfindungsgemäße Verfahren (b) wird vorzugsweise unter Verwendung eines Verdünnungsmittels durchgeführt. Es kommen hierbei die gleichen Verdünnungsmittel in Betracht, die oben für das erfindungsgemäße Verfahren (a) angegeben sind.

Das erfindungsgemäße Verfahren (b) wird vorzugsweise unter Verwendung eines Säureakzeptors durchgeführt. Es kommen hierbei die gleichen Säureakzeptoren in Betracht, die oben für das erfindungsgemäße Verfahren (a) angegeben sind.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Die beim erfindungsgemäßen Verfahren (c) zur Herstellung von Verbindungen der Formel (I) als Ausgangsstoffe zu verwendenden Carbonylverbindungen sind durch die Formel (Ia) allgemein definiert.

In Formel (Ia) haben n, $Q^1$, $Q^3$, $R^1$, $R^2$, $R^3$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $Q^1$, $Q^3$, $R^1$, $R^2$, $R^3$, X, Y und Z angegeben wurden.

Als Beispiele für die Ausgangsstoffe der Formel (Ia) seien genannt:
2-(2-Cyano-ethoxy)-, 2-(3-Cyano-propoxy)-, 2-(1-Carboxy-ethoxy)-, 2-(2-Carboxy-ethoxy)-, 2-(1-Carboxy-propoxy)-, 2-(2-Carboxy-propoxy)-, 2-(3-Carboxy-propoxy)-, 2-(1-Methoxycarbonyl-ethoxy)-, 2-(2-Methoxycarbonyl-ethoxy)-, 2-(1-Methoxycarbonyl-propoxy)-, 2-(2-Methoxycarbonyl-propoxy)- und 2-(3-Methoxycarbonyl-propoxy- -6-(4,6-dimethoxy-pyrimidin-2-yl-oxy)-benzaldehyd.

Die Ausgangsstoffe der Formel (Ia) sind erfindungsgemäße, neue Verbindungen; sie können nach den erfindungsgemäßen Verfahren (a) oder (b) hergestellt werden.

Die beim erfindungsgemäßen Verfahren (c) weiter als Ausgangsstoffe zu verwendenden Aminoverbindungen oder Methylenverbindungen sind durch die Formeln (VI) und (VII) allgemein definiert. In diesen Formeln haben $R^4$, $R^5$ und $R^6$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^4$, $R^5$ und $R^6$ angegeben wurde.

Als Beispiele für die Ausgangsstoffe der Formeln (VI) und (VII) seien genannt:
Ammoniak, Hydroxylamin, Hydrazin, Methylamin, Ethylamin, Propylamin, Isopropylamin, Butylamin, Isobutylamin, sec-Butylamin, tert-Butylamin, Allylamin, Propargylamin, O-Methyl-, O-Ethyl-, O-Propyl-, O-Isopropyl-, O-Butyl-, O-Isobutyl- und O-sec-Butyl-hydroxylamin, O-Allyl-hydroxylamin, Aminooxyessigsäure-methylester und -ethylester, $\alpha$-Aminooxy-propionsäure-methylester und -ethylester, Methylhydrazin, Ethylhydrazin, Propylhydrazin, Isopropylhydrazin, Butylhydrazin, Isobutylhydrazin, sec-Butyl-hydrazin, tert-Butylhydrazin, N,N-Dimethylhydrazin, Acethydrazid, Propionylhydrazld, Methoxycarbonylhydrazin, Ethoxycarbonylhydrazin, Methylsulfonylhydrazin, Ethylsulfonylhydrazin, Phenylhydrazin, Benzoylhydrazin, Benzolsulfonsäurehydrazid, p-Toluolsulfonsäurehydrazid, Malonsäure, Cyanessigsäure, Malonsäuredinitril, Cyanessigsäure-methylester und -ethylester, Malonsäure-dimethylester und -diethylester, $\gamma$-Butyrolacton.

Die Ausgangsstoffe der Formeln (VI) und (VII) sind bekannte Synthesechemikalien.

Das erfindungsgemäße Verfahren (c) wird vorzugsweise unter Verwendung eines Verdünnungsmittels durchgeführt. Es kommen hierbei die gleichen Verdünnungsmittel in Betracht, die oben für das erfindungsgemäße Verfahren (a) angegeben sind.

Das erfindungsgemäße Verfahren (c) wird gegebenenfalls in Gegenwart eines Reaktionshilfsmittels durchgeführt. Geeignete Reaktionshilfsmittel sind Stoffe, die üblicherweise zur Steuerung und/oder Beschleunigung von Kondensationsreaktionen zwischen Carbonylverbindungen und Amino- oder Methylen-Verbindungen verwendet werden. Hierzu gehören insbesondere basische Verbindungen, wie z.B. Natriumacetat, Ammoniumacetat, $\beta$-Alanin, Pyridin und Piperidin.

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 10 °C und 120 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vergleiche die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Öilpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur Bekämpfung von Unkräutern im Vorauflauf- und im Nachauflauf-Verfahren.

In gewissem Umfang zeigen sie auch fungizide Wirkung, beispielsweise gegen Pyricularia oryzae.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktlonierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester,

Polyoxyethylen-Fettalkohol-Ether, z.B. Alkylaryl-polyglykol-ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb-stoffe und Spurennährstoffe wie Salze von Eisen, Mangan, Bor, Kupfer, Kobalt, Molybdän und Zink verwendet werden.

Die Formulierungen enthalten Im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzini; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertlge Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

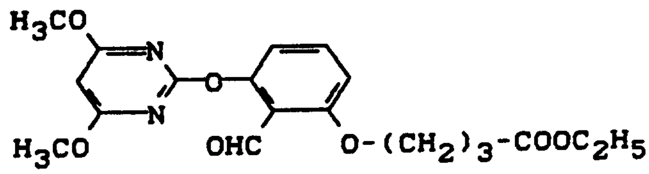

(Verfahren (a))

Eine Mischung aus 1,66 g (6 mMol) 2-Hydroxy-6-(4,6-dimethoxy-pyrimidin-2-yl-oxy)-benzaldehyd, 1,17 g (6 mMol) γ-Brom-buttersäureethylester, 2,1 g (15 mMol) Kaliumcarbonat und 50 ml Acetonitril wird 8 Stunden unter Rückfluß erhitzt und dann im Vakuum eingeengt. Der Rückstand wird mit Essigsäureethylester/Wasser geschüttelt, die organische Phase mit gesättigter Natriumhydrogencarbonat-Lösung gewaschen, mit Natriumsulfat getrocknet und filtriert. Das Filtrat wird im Vakuum eingeengt, der Rückstand mit Diethylether disgeriert und das kristalline Produkt durch Absaugen isoliert.

Man erhält 1,2 g (51% der Theorie) 2-(3-Ethoxycarbonylpropoxy)-6-(4,6-dimethoxy-pyrimidin-2-γl-oxy)-benzaldehyd vom Schmelzpunkt 96 °C.

Beispiel 2

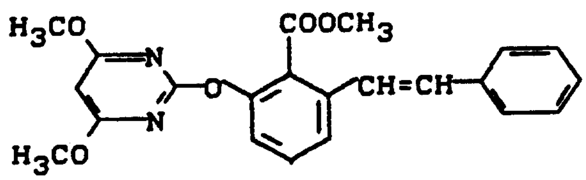

(Verfahren (b))

Eine Mischung aus 1,2 g (4,7 mMol) 2-Hydroxy-6-styrylbenzoesäuremethylester, 1,02 g (4,7 mMol) 4,6-Dimethoxy-2-methylsulfonyl-pyrimidin, 0,8 g (5,7 mMol) Kaliumcarbonat und 50 ml Acetonitril wird 15 Stunden unter Rückfluß erhitzt und dann eingeengt. Der Rückstand wird mit Methylenchlorid/Wasser geschüttelt, die organische Phase mit Natriumsulfat getrocknet und filtriert. Das Filtrat wird eingeengt und der Rückstand durch Säulenchromatographie (Kieselgel; Hexan/Essigsäureethylester, 10:1) gereinigt.

Man erhält 1,0 g (54% der Theorie) 2-Styryl-6-(4,6-dimethoxy-pyrimidin-2-yl-oxy)-benzoesäuremethylester als amorphes Produkt.

Beispiel 3

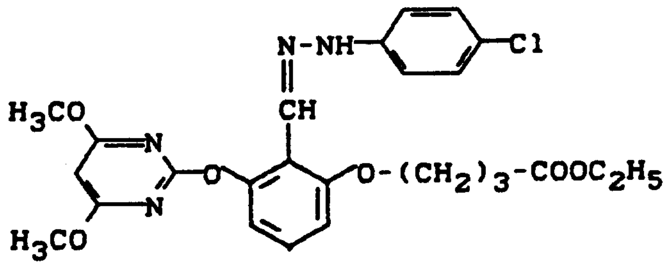

(Verfahren (c))

Eine Mischung aus 2,93 g (7,5 mMol) 2-(3-Ethoxycarbonylpropoxy)-6-(4,6-dimethoxy-pyrimidin-2-yl-oxy)-benzaldehyd (vgl. Beispiel 1), 1,5 g (8,3 mMol) 4-Chlor-phenylhydrazin-hydrochlorid, 0,76 g (9,3 mMol) Natriumacetat und 150 ml Methylenchlorid wird 15 Stunden bei 20 °C gerührt, dann mit gesättigter Dinatriumhydrogenphosphat-Lösung gewaschen, mit Natriumsulfat getrocknet und filtriert. Das Filtrat wird eingeengt, der Rückstand mit Methanol verrührt und das kristalline Produkt durch Absaugen isoliert.

Man erhält 2,6 g (67% der Theorie) 2-(3-Ethoxycarbonylpropoxy)-6-(4,6-dimethoxy-pyrimidin-2-yl-oxy)-benzaldehyd-4-chlorphenylhydrazon vom Schmelzpunkt 139 °C.

Analog zu den Beispielen 1 bis 3 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

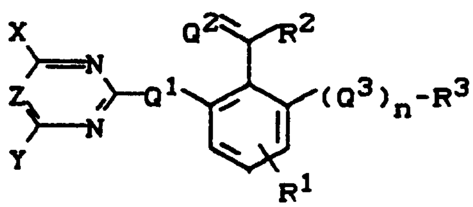

(I)

**Tabelle 1:** Beispiele für die Verbindungen der Formel (I)

| Bsp.-Nr. | n | $Q^1$ | $Q^2$ | $Q^3$ | $R^1$ | $R^2$ | $R^3$ | X | Y | Z | Schmelzpunkt (°C) |
|---|---|---|---|---|---|---|---|---|---|---|---|
| 4 | 1 | O | N-NH-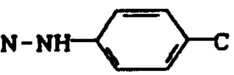-Cl | O | H | H | $-CHCOOCH_3$<br>$CH_3$ | $OCH_3$ | $OCH_3$ | CH | 158 |
| 5 | 0 | 0 | O | - | (3-)Br | OH | -CH=CH-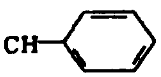 | $OCH_3$ | $OCH_3$ | CH | |
| 6 | 0 | 0 | O | - | H | OH | -CH=CH-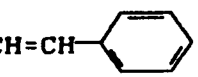 | $OCH_3$ | $OCH_3$ | CH | |
| 7 | 0 | 0 | O | - | H | OH | -CH=CH-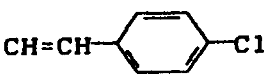-Cl | $OCH_3$ | $OCH_3$ | CH | 128 |
| 8 | 0 | 0 | O | - | H | $OCH_3$ | -CH=CH-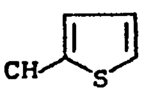 | $OCH_3$ | $OCH_3$ | CH | 113 |
| 9 | 0 | 0 | O | - | H | $OCH_3$ | -CH=C-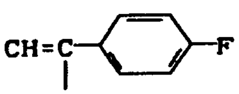-F<br>$CH_3$ | $OCH_3$ | $OCH_3$ | CH | |
| 10 | 0 | 0 | O | - | H | $OCH_3$ | -CH=C-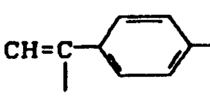-$OCH_3$<br>$CH_3$ | $OCH_3$ | $OCH_3$ | CH | |

Tabelle 1 - Fortsetzung

| Bsp.-Nr. | n | $Q^1$ | $Q^2$ | $Q^3$ | $R^1$ | $R^2$ | $R^3$ | X | Y | Z | Schmelzpunkt (°C) |
|---|---|---|---|---|---|---|---|---|---|---|---|
| 11 | 0 | 0 | 0 | - | H | OH | -CH=CH 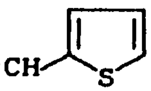 | $OCH_3$ | $OCH_3$ | CH | |
| 12 | 0 | 0 | 0 | - | H | $OCH_3$ | 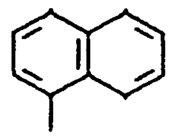 CH=CH- | $OCH_3$ | $OCH_3$ | CH | |
| 13 | 0 | 0 | 0 | - | H | OH | 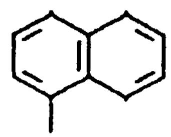 CH=CH- | $OCH_3$ | $OCH_3$ | CH | |
| 14 | 0 | 0 | 0 | - | H | OH | -CH=CH 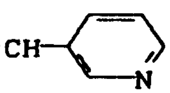 | $OCH_3$ | $OCH_3$ | CH | |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzontration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 2 und 6 starke Wirkung gegen Unkräuter.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 2, 5 und 6 starke Wirkung gegen Unkräuter.

**Patentansprüche**

**1.** Substituierte Azine der allgemeinen Formel (I)

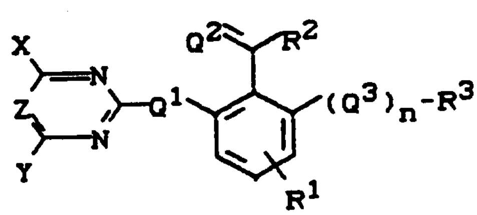

(I)

dadurch gekennzeichnet, daß

| | |
|---|---|
| n | für die Zahlen 0 oder 1 steht, |
| $Q^1$ und $Q^3$ | gleich oder verschieden sind und für Sauerstoff, Schwefel, NH oder N-Alkyl stehen, |
| $Q^2$ | für Sauerstoff, Schwefel oder eine der nachstehenden Gruppierungen steht: N-$R^4$ oder |

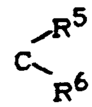

$R^1$ für Wasserstoff, Amino, Hydroxy, Cyano, Nitro, Halogen oder für einen Rest der Reihe Alkyl, Halogenalkyl, Alkoxy, Halogenalkoxy, Alkylthio, Halogenalkylthio, Alkylamino, Dialkylamino, Alkylcarbonylamino, Alkoxycarbonylamino, Alkylsulfonylamino oder Phenyl steht,

$R^2$ für Wasserstoff, Hydroxy, Alkyl oder für einen jeweils gegebenenfalls substituierten Rest der Reihe Alkoxy, Alkylthio, Alkylamino, Dialkylamino, Aralkyloxy, Aralkylthio, Aralkylamino, Aryloxy, Arylthio oder Arylamino steht,

$R^3$ für durch Cyano, Carboxy oder Alkoxycarbonyl substituiertes Alkyl mit mindestens zwei Kohlenstoffatomen oder für gegebenenfalls durch Cyano, Carboxy, Halogen, Alkoxycarbonyl, Aryl (welches gegebenenfalls durch Halogen, Alkyl oder Alkoxy substituiert ist) oder Heteroaryl substituiertes Alkenyl steht,

$R^4$ für Wasserstoff, Amino oder für einen jeweils gegebenenfalls substituierten Rest der Reihe Alkyl, Alkenyl, Alkinyl, Aralkyl, Aryl, Alkoxycarbonyloxy, Arylaminocarbonyloxy, Carboxyalkoxy, Alkoxycarbonylalkoxy, Alkylamino, Dialkylamino, Aralkylamino, Arylamino, N-Alkyl-N-arylamino, Alkylcarbonylamino, Arylcarbonylamino, Heteroarylamino, Heteroarylcarbonylamino, Alkoxycarbonylamino, Alkylsulfonylamino oder Arylsulfonylamino steht,

$R^5$ für Wasserstoff, Halogen, Cyano, Carboxy, Alk-oxycarbonyl, Alkylcarbonylamino oder Dialkoxyphosphoryl steht,

$R^6$ für Formyl, Cyano, Carboxy, Hydroxymethyl, Carbamoyl oder für einen jeweils gegebenenfalls substituierten Rest der Reihe Alkoxycarbonyl, Cycloalkyloxycarbonyl, Aralkoxycarbonyl, Aryloxycarbonyl, Heterocyclylalkoxycarbonyl, Alkylthiocarbonyl, Aralkylthiocarbonyl, Arylthiocarbonyl, Alkylaminocarbonyl` Cycloalkylaminocarbonyl, Aralkylaminocarbonyl, Arylaminocarbonyl, Dialkylaminocarbonyl, Arylaminocarbonylalkoxycarbonyl, Dialkylaminocarbonylalkoxycarbonyl, Alkylaminocarbonylalkoxycarbonyl, Alkylhydrazinocarbonyl, Arylhydrazinocarbonyl, Pyrrolidinylcarbonyl, Piperidinylcarbonyl, Morpholinylcarbonyl, Piperazinylcarbonyl oder Phthalimidoxycarbonyl steht,

X und Y gleich oder verschieden sind und für Wasserstoff, Halogen oder einen Rest der Reihe Alkyl, Halogenalkyl, Alkoxy, Halogenalkoxy, Alkylthio, Alkylamino oder Dialkylamino stehen und

Z für Stickstoff oder eine CH- oder C-Halogen-Gruppierung steht.

**2.** Substituierte Azine der allgemeinen Formel (I) gemäß Anspruch 1, dadurch gekennzeichnet, daß

n für die Zahlen 0 oder 1 steht,

$Q^1$ und $Q^3$ gleich oder verschieden sind und für Sauerstoff, Schwefel, NH oder N-$C_1$-$C_4$-Alkyl stehen,

$Q^2$ für Sauerstoff, Schwefel oder eine der nachstehenden Gruppierungen steht:
N-$R^4$ oder

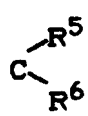

$R^1$ für Wasserstoff, Amino, Hydroxy, Cyano, Nitro, Halogen oder für einen Rest der Reihe $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Di-($C_1$-$C_4$-alkyl)-amino, $C_1$-$C_4$-Alkyl-carbonylamino, $C_1$-$C_4$-Alkoxy-carbonylamino, $C_1$-$C_4$-Alkylsulfonylamino oder Phenyl steht,

$R^2$ für Wasserstoff, Hydroxy, $C_1$-$C_4$-Alkyl oder für einen jeweils gegebenenfalls substituierten Rest der Reihe $C_1$-$C_6$-Alkoxy, $C_1$-$C_6$-Alkylthio, $C_1$-$C_6$-Alkylamino, Di-($C_1$-$C_4$-

alkyl)-amino, Phenyl-$C_1$-$C_2$-alkyloxy, Phenyl-$C_1$-$C_2$-alkylthio, Phenyl-$C_1$-$C_2$-alkylamino, Phenoxy, Phenylthio oder Phenylamino steht, wobei die möglichen Substituenten in den Alkylteilen vorzugsweise ausgewählt sind aus der Reihe Fluor, Chlor, Cyano, Nitro, Carboxy, $C_1$-$C_4$-Alkoxy, $C_1$-$C_4$-Alkoxy-carbonyl, und die möglichen Substituenten in den Arylteilen vorzugsweise ausgewählt sind aus der Reihe Fluor, Chlor, Brom, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxy-carbonyl,

$R^3$ für durch Cyano, Carboxy oder $C_1$-$C_4$-Alkoxycarbonyl substituiertes $C_2$-$C_6$-Alkyl oder für gegebenenfalls durch Cyano, Carboxy, Halogen, $C_1$-$C_4$-Alkoxy-carbonyl, Phenyl (welches gegebenenfalls durch Halogen, $C_1$-$C_4$-Alkyl oder $C_1$-$C_4$-Alkoxy substituiert ist), Naphthyl, Thienyl, Furyl oder Pyridyl substituiertes $C_2$-$C_6$-Alkenyl steht,

$R^4$ für Wasserstoff, Amino oder für einen jeweils gegebenenfalls durch Halogen substituierten Rest der Reihe $C_1$-$C_6$-Alkyl, $C_3$-$C_6$-Alkenyl, $C_3$-$C_6$-Alkinyl, Carboxy-$C_1$-$C_2$-alkoxy, $C_1$-$C_6$-Alkoxycarbonyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkyl-carbonylamino, $C_1$-$C_6$-Alkoxy-carbonylamino, $C_1$-$C_6$-Alkylsulfonylamino, oder für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenylaminocarbonyloxy, Phenylamino, Phenyl-$C_1$-$C_4$-alkylamino, N-($C_1$-$C_4$-alkyl)-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^5$ für Wasserstoff, Halogen, Cyano, Carboxy, $C_1$-$C_6$-Alkoxy-carbonyl, $C_1$-$C_6$-Alkylcarbonylamino oder Di-($C_1$-$C_4$-alkoxy)-phosphoryl steht,

$R^6$ für Formyl, Cyano, Carboxy, Hydroxymethyl, Carbamoyl oder für einen jeweils gegebenenfalls durch Halogen, Carboxy oder $C_1$-$C_4$-Alkoxy-carbonyl substituierten Rest der Reihe $C_1$-$C_6$-Alkoxy-carbonyl, $C_5$-$C_6$-Cycloalkyloxy-carbonyl, $C_1$-$C_6$-Alkylthio-carbonyl, $C_1$-$C_6$-Alkylamino-carbonyl, $C_5$-$C_6$-Cycloalkylamino-carbonyl, Di($C_1$ $C_2$-alkyl)-amino-carbonyl, $C_1$-$C_4$-Alkylamino-carbonyl-$C_1$-$C_4$-alkoxy-carbonyl, Di-($C_1$-$C_2$-alkyl)-amino-carbonyl-$C_1$-$C_4$-alkoxy-carbonyl, Phenylaminocarbonyl-$C_1$-$C_4$-alkoxy-carbonyl, N-Methyl-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di($C_1$-$C_2$-alkyl)-amino substituiertes Phenoxycarbonyl, Phenyl-$C_1$-$C_4$-alkoxycarbonyl, Furylmethoxycarbonyl, Thienylmethoxycorbonyl, Phenylthiocarbonyl, Phenyl-$C_1$-$C_4$-alkylthiocarbonyl, Phenylsminocarbonyl, Phenyl-$C_1$-$C_4$-alxylamino-carbonyl, N-($C_1$-$C_4$-Alkyl)-phenylamino-carbonyl oder Phenylhydrazinocarbonyl oder für Phthalimidoxycarbonyl steht,

X und Y gleich oder verschieden sind und für Wasserstoff, Halogen oder einen Rest der Reihe $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen und

Z für Stickstoff oder eine CH oder C-Halogen-Gruppierung steht.

**3.** Substituierte Azine der allgemeinen Formel (I) gemaß Anspruch 1, dadurch gekennzeichnet, daß

n für die Zahlen 0 oder 1 steht,

$Q^1$ und $Q^3$ jeweils für Sauerstoff stehen,

$Q^2$ für Sauerstoff oder eine der nachstehenden Gruppierungen steht:

N-$R^4$ oder

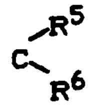

$R^1$ für Wasserstoff, Amino, Hydroxy, Fluor, Chlor, Brom, Methyl, Trifluormethyl, Methoxy,

Difluormethoxy, Trifluormethoxy, Methylthio, Trifluormethylthio, Methylamino, Dimethylamino, Acetylamino, Methoxycarbonylamino, Methylsulfonylamino oder Phenyl steht,

$R^2$ für Wasserstoff, Hydroxy, Methyl oder für jeweils gegebenenfalls substituiertes Methoxy, Ethoxy, Propoxy, Butoxy, Benzyloxy, Phenylethoxy oder Phenoxy steht, wobei die möglichen Substituenten in den Alkylteilen vorzugsweise ausgewählt sind aus der Reihe Fluor, Chlor, Cyano, Carboxy, Methoxy, Ethoxy, Methoxycarbonyl, Ethoxycarbonyl, und die möglichen Substituenten in den Arylteilen vorzugsweise ausgewählt sind aus der Reihe Fluor, Chlor, Brom, Methyl, Trifluormethyl, Methoxy, Trifluormethoxy, Methoxycarbonyl und Ethoxycarbonyl,

$R^3$ für durch Cyano, Carboxy, Methoxycarbonyl oder Ethoxycarbonyl substituiertes Ethyl, Propyl oder Butyl oder für gegebenenfalls durch Cyano, Carboxy, Fluor, Chlor, Brom, Methoxycarbonyl, Ethoxycarbonyl, Phenyl (welches gegebenenfalls durch Fluor, Chlor, Brom, Methyl oder Methoxy substituiert ist), Naphthyl, Thienyl, Furyl oder Pyridyl substituiertes Ethenyl, Propenyl oder Butenyl steht,

$R^4$ für Wasserstoff, Amino, für Methyl, Ethyl, Propyl, Isopropyl, Butyl, Isobutyl, sec-Butyl, tert-Butyl, Allyl, Propargyl, Carboxymethoxy, Carboxyethoxy, Methoxycarbonyloxy, Ethoxycarbonyloxy, Propoxycarbonyloxy, Butoxycarbonyloxy, Methoxycarbonylmethoxy, Ethoxycarbonylmethoxy, Methoxycarbonylethoxy, Ethoxycarbonylethoxy, Methylamino, Ethylamino, Propylamino, Isopropylamino, Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenyl, Benzyl, Phenylaminocarbonyloxy, Phenylamino, Benzylamino, N-Methyl-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^5$ für Wasserstoff, Fluor, Chlor, Cyano, Carboxy, $C_1$-$C_4$-Alkoxy-carbonyl, $C_1$-$C_4$-Alkylcarbonylamino, Dimethoxyphosphoryl oder Diethoxyphosphoryl steht,

$R^6$ für Formyl, Cyano, Carboxy, Hydroxymethyl, Carbamoyl, für einen jeweils gegebenenfalls durch Fluor, Chlor, Carboxy oder $C_1$-$C_4$-Alkoxy-carbonyl substituierten Rest der Reihe $C_1$-$C_4$-Alkoxy-carbonyl, $C_5$-$C_6$-Cycloalkyloxycarbonyl, $C_1$-$C_4$-Alkylthiocarbonyl, $C_1$-$C_4$-Alkylamino-carbonyl, $C_5$-$C_6$-Cycloalkylamino-carbonyl, Dimethylaminocarbonyl, $C_1$-$C_4$-Alkylaminocarbonyl-$C_1$-$C_4$-alkoxycarbonyl, für Dimethylaminocarbonyl-$C_1$-$C_4$-alkoxy-carbonyl, für N-Methylphenylaminocarbonyl-$C_1$-$C_4$-alkoxycarbonyl, für jeweils gegebenenfalls durch Methyl und/oder Ethyl substituiertes Pyrrolidinyl-carbonyl, Piperidinyl-carbonyl, Morpholinylcarbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-phenylaminocarbonyl, Phenylhydrazinocarbonyl oder für Phthalimidoxycarbonyl steht,

X und Y gleich oder verschieden sind und für Wasserstoff, Chlor, Methyl, Ethyl, Trifluormethyl, Methoxy, Ethoxy, Difluormethoxy, Methylthio, Methylamino, Ethylamino oder Dimethylamino stehen und

Z für Stickstoff oder eine CH-Gruppierung steht.

**4.** Verfahren zur Herstellung substituierter Azine der allgemeinen Formel (I) gemäß Anspruch 1,

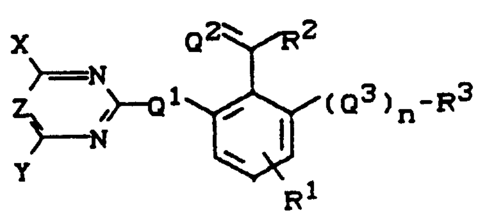

(I)

in welcher

n für die Zahlen 0 oder 1 steht,

$Q^1$ und $Q^3$ gleich oder verschieden sind und für Sauerstoff, Schwefel, NH oder N-Alkyl stehen,

$Q^2$ für Sauerstoff, Schwefel oder eine der nachstehenden Gruppierungen steht:

N-$R^4$ oder

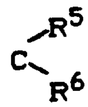

$R^1$ für Wasserstoff, Amino, Hydroxy, Cyano, Nitro, Halogen oder für einen Rast der Reihe Alkyl, Halogenalkyl, Alkoxy, Halogenalkoxy, Alkylthio, Halogenalkylthio, Alkylamino, Dialkylamino, Alkylcarbonylamino, Alkoxycarbonylamino, Alkylsulfonylamino oder Phenyl steht,

$R^2$ für Wasserstoff, Hydroxy, Alkyl oder für einen jeweils gegebenenfalls substituierten Rest der Reihe Alkoxy, Alkylthio, Alkylamino, Dialkylamino, Aralkyloxy, Aralkylthio, Aralkylamino, Aryloxy, Arylthio oder Arylamino steht,

$R^3$ für durch Cyano, Carboxy oder Alkoxycarbonyl substituiertes Alkyl mit mindestens zwei Kohlenstoffatomen oder für gegebenenfalls durch Cyano, Carboxy, Halogen, Alkoxycarbonyl, Aryl (welches gegebenenfalls durch Halogen, Alkyl oder Alkoxy substituiert ist) oder Heteroaryl substituiertes Alkenyl steht,

$R^4$ für Wasserstoff, Amino oder für einen jeweils gegebenenfalls substituierten Rest der Reihe Alkyl, Alkenyl, Alkinyl, Aralkyl, Aryl, Alkoxycarbonyloxy, Arylaminocarbonyloxy, Carboxyalkoxy, Alkoxycarbonylalkoxy, Alkylamino, Dialkylamino, Aralkylamino, Arylamino, N-Alkyl-N-arylamino, Alkylcarbonylamino, Arylcarbonylamino, Heteroarylamino, Heteroarylcarbonylamino, Alkoxycarbonylamino, Alkylsulfonylamino oder Arylsulfonylamino steht,

$R^5$ für Wasserstoff, Halogen, Cyano, Carboxy, Alk-oxycarbonyl, Alkylcarbonylamino oder Dialkoxyphosphoryl steht,

$R^6$ für Formyl, Cyano, Carboxy, Hydroxymethyl, Carbamoyl oder für einen jeweils gegebenenfalls substituierten Rest der Reihe Alkoxycarbonyl, Cycloalkyloxycarbonyl, Aralkoxycarbonyl, Aryloxycarbonyl, Heterocyclylalkoxycarbonyl, Alkylthiocarbonyl, Aralkylthiocarbonyl, Arylthiocarbonyl, Alxylaminocarbonyl, Cycloalkylaminocarbonyl, Aralkylaminocarbonyl, Arylaminocarbonyl, Dialxylaminocarbonyl, Arylaminocarbonylalxoxycarbonyl, Dialkylaminocarbonylalkoxycarbonyl, Alkylaminocarbonylalkoxycarbonyl, Alkylhydrszinocarbonyl, Arylhydrazinocarbonyl, Pyrrolidinylcarbonyl, Piperidinylcarbonyl, Morpholinylcarbonyl, Piperazinylcarbonyl oder Phthalimidoxycarbonyl steht,

X und Y gleich oder verschieden sind und für Wasserstoff, Halogen oder einen Rest der Reihe Alkyl, Halogenalkyl, Alkoxy, Halogenalkoxy, Alkylthio, Alkylamino oder Dialkylamino stehen und

Z für Stickstoff oder eine CH- oder C-Halogen-Gruppierung steht,

dadurch gekennzeichnet, daß man

(a) Azinverbindungen der allgemeinen Formel (II)

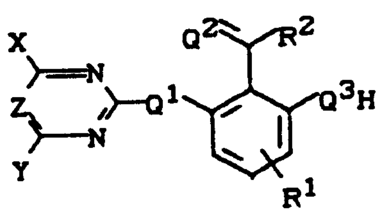

(II)

in welcher

$Q^1$, $Q^2$, $Q^3$, $R^1$, $R^2$, X, Y und Z die oben angegebenen Bedeutungen haben,

mit Halogenverbindungen der allgemeinen Formel) (III

$X^1$-$R^3$ (III)

in welcher
$R^3$ die oben angegebenen Bedeutungen hat und
$X^1$ für Halogen steht,
gegebenenfalls in Gegenwart eines Säureakzeptors und gegebenenfalls in Gegenwart eines Verdünnungsmittels umsetzt, oder daß man
(b) Benzolderivate der allgemeinen Formel (IV)

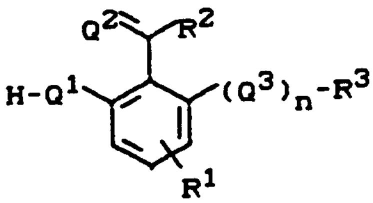

(IV)

in welcher
n, $Q^1$, $Q^2$, $Q^3$, $R^1$, $R^2$ und $R^3$ die oben angegebenen Bedeutungen haben,
mit reaktionsfähigen Azinen der allgemeinen Formel (V)

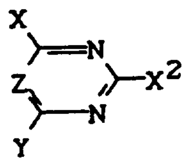

(V)

in welcher
X, Y und Z die oben angegebene Bedeutung haben und
$X^2$ für eine nucleophile Abgangsgruppe steht,
gegebenenfalls in Gegenwart eines Säureakzeptors und gegebenenfalls in Gegenwart eines Verdünnungsmittels umsetzt, oder daß man
(c) Carbonylverbindungen der allgemeinen Formel (Ia)

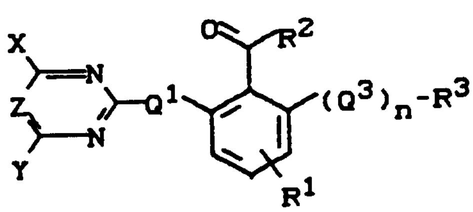

(Ia)

in welcher
n, $Q^1$, $Q^3$, $R^1$, $R^2$, $R^3$, X, Y und Z die oben angegebenen Bedeutungen haben,
mit Aminoverbindungen der allgemeinen Formel (VI)

$H_2N$-$R^4$ (VI)

in welcher
$R^4$ die oben angegebenen Bedeutungen hat,
oder mit Methylenverbindungen der allgemeinen Formel (VII)

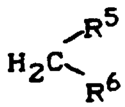

(VII)

in welcher

$R^5$ und $R^6$ die oben angegebenen Bedeutungen haben,

gegebenenfalls in Gegenwart eines Reaktionshilfsmittels und gegebenenfalls in Gegenwart eines Verdünnungsmittels umsetzt.

**5.** Herbizide Mittel, gekennzeichnet durch einen Gehalt an mindestens einem substituierten Azin der Formel (I) gemäß den Ansprüchen 1 bis 4.

**6.** Verfahren zur Bekämpfung von unerwünschten Pflanzen, dadurch gekennzeichnet, daß man substituierte Azine der Formel (I) gemäß den Ansprüchen 1 bis 4 auf unerwünschte Pflanzen und/oder ihren Lebensraum einwirken läßt.

**7.** Verwendung von substituierten Azinen der Formel (I) gemäß den Ansprüchen 1 bis 4 zur Bekämpfung von unerwünschten Pflanzen.

**8.** Verfahren zur Herstellung von herbiziden Mitteln, dadurch gekennzeichnet, daß man substituierte Azine der Formel (I) gemäß den Ansprüchen 1 bis 4 mit Streckmitteln und/oder oberflächenaktiven Substanzen vermischt.

**9.** Carbonylverbindungen der allgemeinen Formel (Ia),

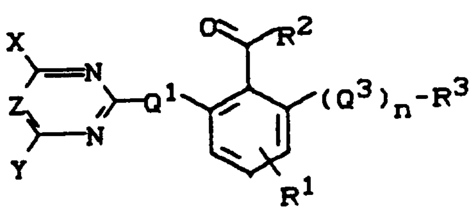

(Ia)

dadurch gekennzeichnet, daß

| | |
|---|---|
| n | für die Zahlen 0 oder 1 steht, |
| $Q^1$ und $Q^3$ | gleich oder verschieden sind und für Sauerstoff, Schwefel, NH oder N-Alkyl stehen, |
| $R^1$ | für Wasserstoff, Amino, Hydroxy, Cyano, Nitro, Halogen oder für einen Rest der Reihe Alkyl, Halogenalkyl, Alkoxy, Halogenalkoxy, Alkylthio, Halogenalkylthio, Alkylamino, Dialkylamino, Alkylcarbonylamino, Alkoxycarbonylamino, Alkylsulfonylamino oder Phenyl steht, |
| $R^2$ | für Wasserstoff, Hydroxy, Alkyl oder für einen jeweils gegebenenfalls substituierten Rest der Reihe Alkoxy, Alkylthio, Alkylamino, Dialkylamino, Aralkyloxy, Aralkylthio, Aralkylamino, Aryloxy, Arylthio oder Arylamino steht, |
| $R^3$ | für durch Cyano, Carboxy oder Alkoxycarbonyl substituiertes Alkyl mit mindestens zwei Kohlenstoffatomen oder für gegebenenfalls durch Cyano, Carboxy, Halogen, Alkoxycarbonyl, Aryl (welches gegebenenfalls durch Halogen, Alkyl oder Alkoxy substituiert ist) oder Heteroaryl substituiertes Alkenyl steht, |
| X und Y | gleich oder verschieden sind und für Wasserstoff, Halogen oder einen Rest der Reihe Alkyl, Halogenalkyl, Alkoxy, Halogenalkoxy, Alkylthio, Alkylamino oder Dialkylamino stehen und |
| Z | für Stickstoff oder eine CH- oder C-Halogen-Gruppierung steht. |

Europäisches Patentamt

# EUROPÄISCHER RECHERCHENBERICHT

Nummer der Anmeldung

EP 93 10 4985

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X,D | EP-A-0 459 243 (BAYER AG)<br>* das ganze Dokument * | 1,5 | C07D239/34<br>C07D239/60<br>C07D251/30<br>A01N43/54<br>A01N43/66 |
| A | EP-A-0 469 711 (SUMITOMO CHEMICAL COMPANY,LIMITED)<br>* Seite 4 - Seite 6, Zeile 32 * | 1,5 | |
| A | EP-A-0 402 751 (BASF AKTIENGESELLSCHAFT)<br>* Seite 3 - Seite 5, Zeile 45 * | 1,5 | |
| A | EP-A-0 346 789 (BASF AKTIENGESELLSCHAFT)<br>* Seite 3 - Seite 4, Zeile 56 * | 1,5 | |
| A,D | EP-A-0 287 079 (KUMIAI CHEMICAL INDUSTRY CO.)<br>* Seite 3 - Seite 5 * | 1,5 | |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| BERLIN | 13 JULI 1993 | KYRIAKAKOU G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)